# EUROPEAN PATENT APPLICATION

(11) **EP 4 350 709 A1**
(43) Date of publication of application: **10.04.2024**
(21) Application number: 23200963.9
(22) Date of filing: 29.09.2023
(51) Int. Cl.: G16H 50/30, G16H 50/20, G16H 50/50

(54) **METHOD AND SYSTEM FOR IDENTIFYING UNHEALTHY BEHAVIOR TRIGGER AND PROVIDING NUDGES**

(30) Priority: 07.10.2022 IN 202221057587
(71) Applicant: Tata Consultancy Services Limited, Maharashtra (IN)
(72) Inventor: CHANDEL, VIVEK, 201301 Noida, Uttar Pradesh (IN); GHOSE, AVIK, 700160 Kolkata, West Bengal (IN); DUGGIRALA, MAYURI, 411057 Pune, Maharashtra (IN); CHATTERJEE, ARNAB, 201309 Noida, Uttar Pradesh (IN); BHATTACHARYA, SAKYAJIT, 700160 Kolkata, West Bengal (IN)
(74) Representative: Goddar, Heinz J.

(57) **Abstract**

Existing systems for behavioural tracking and identification have the disadvantage that they do not analyse data in behavioural aspects. As a result, they lack ability to preempt scenarios involving actions that adversely affect user health. The disclosure herein generally relates to behavior prediction, and, more particularly, to a method and system for identifying unhealthy behavior trigger and providing nudges. The system generates a casual inference model, which is a reverse causality model facilitating mapping of context with one or more behaviour of the user. The system further collects and processes real-time data using the casual inference model, to perform behavioral analysis of the user.

## Description

### CROSS-REFERENCE TO RELATED APPLICATIONS AND PRIORITY

The present application claims priority to Indian application no. 202221057587, filed on October 07, 2022.

### TECHNICAL FIELD

The disclosure herein generally relates to behavior prediction, and, more particularly, to a method and system for identifying unhealthy behavior trigger and providing nudges.

### BACKGROUND

Unhealthy habits such as but not limited to smoking, and alcohol consumption have an adverse impact on health of a person. Some people may start such habits due to impact of the environment they are in. For example, a person who spends time with another person who is an addict to one or more such habits has a higher chance/probability of getting exposed to such habits and in turn becoming an addict. In such instances, the situations/context have an impact on the behaviour of the user.

Existing systems for behavioural tracking and identification have the disadvantage that they do not analyse data in behavioural aspects. As a result, they lack ability to pre-empt such scenarios from happening.

### SUMMARY

Embodiments of the present disclosure present technological improvements as solutions to one or more of the above-mentioned technical problems recognized by the inventors in conventional systems. For example, in one embodiment, a processor implemented method is provided. The method includes fetching, via one or more hardware processors, a context time-series data and a health behaviour time-series data, as input data, wherein the context time-series data includes context information related to a user, and the health behaviour time-series data includes information on a plurality of health parameters of the user collected over a period of time. Further, correlation between the context time-series data and a health behaviour time-series data is determined via the one or more hardware processors. Further, a relationship data comprising information on one or more contexts in the context time-series data is determined as causing one or more health conditions in the health behaviour time-series data, via the one or more hardware processors. Further, a casual inference model is generated using the relationship data, via the one or more hardware processors.

In another aspect, the method further comprising obtaining context specific information pertaining to a user being monitored. Further, one or more health conditions corresponding to the context specific information are determined. Further, it is determined whether the determined one or more health conditions belong to a risk category, by processing the context specific information and the one or more health conditions using the casual inference model. Further, one or more nudges are provided to the user, if the determined one or more health conditions belong to the risk category, to avert one or more actions determined as adversely affecting health of the user, from happening.

In yet another aspect, a system is provided. The system includes one or more hardware processors, a communication interface, and a memory storing a plurality of instructions. The plurality of instructions when executed, cause the one or more hardware processors to fetch a context time-series data and a health behaviour time-series data, as input data, wherein the context time-series data includes context information related to a user, and the health behaviour time-series data includes information on a plurality of health parameters of the user collected over a period of time. Further, correlation between the context time-series data and a health behaviour time-series data is determined via the one or more hardware processors. Further, a relationship data comprising information on one or more contexts in the context time-series data is determined as causing one or more health conditions in the health behaviour time-series data, via the one or more hardware processors. Further, a casual inference model is generated using the relationship data, via the one or more hardware processors.

In yet another aspect, the system is configured to obtain context specific information pertaining to a user being monitored. The system determines one or more health conditions corresponding to the context specific information. The system further determines whether the determined one or more health conditions belong to a risk category, by processing the context specific information and the one or more health conditions using the casual inference model, and provides one or more nudges to the user, if the determined one or more health conditions belong to the risk category, to avert one or more actions determined as adversely affecting health of the user, from happening.

In yet another aspect, a non-transitory computer readable medium is provided. The non-transitory computer readable medium includes a plurality of instructions, which when executed, cause the one or more hardware processors to fetch a context time-series data and a health behaviour time-series data, as input data, wherein the context time-series data includes context information related to a user, and the health behaviour time-series data includes information on a plurality of health parameters of the user collected over a period of time. Further, correlation between the context time-series data and a health behaviour time-series data is determined via the one or more hardware processors. Further, a relationship data comprising information on one or more contexts in the context time-series data is determined as causing one or more health conditions in the health behaviour time-series data, via the one or more hardware processors. Further, a casual inference model is generated using the relationship data, via the one or more hardware processors.

In another aspect, the plurality of instructions in the non-transitory computer readable medium further cause the one or more hardware processors to obtain a context specific information pertaining to a user being monitored. Further, one or more health conditions corresponding to the context specific information are determined. Further, it is determined whether the determined one or more health conditions belong to a risk category, by processing the context specific information and the one or more health conditions using the casual inference model. Further, one or more nudges are provided to the user, if the determined one or more health conditions belong to the risk category, to avert one or more actions determined as adversely affecting health of the user, from happening.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for behavioral assessment of a user, according to some embodiments of the present disclosure.
FIG. 2 is a flow diagram depicting steps involved in the process of generating a causal inference model for behavioral assessment of diagram according to some embodiments of the present disclosure.
FIG. 3 is a flow diagram depicting steps involved in the process of performing the behavioral assessment of a user, using the causal inference model, by the system of FIG. 1, in accordance with some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Unhealthy habits such as but not limited to smoking, and alcohol consumption have an adverse impact on health of a person. Some people may start such habits due to impact of the environment they are in. For example, a person who spends time with another person who is an addict to one or more such habits has a higher chance/probability of getting exposed to such habits and in turn becoming an addict. In such instances, the situations/context have an impact on the behaviour of the user. Existing systems for behavioural tracking and identification have the disadvantage that they do not analyse data in behavioural aspects. As a result, they lack ability to pre-empt such scenarios from happening.

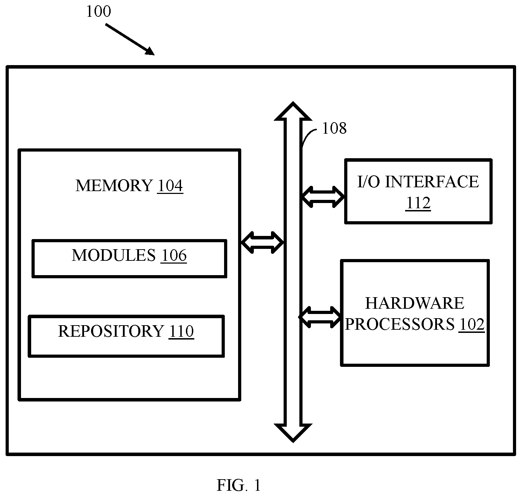

Referring now to the drawings, and more particularly to FIG. 1 through FIG. 3, where similar reference characters denote corresponding features consistently throughout the figures, there are shown preferred embodiments and these embodiments are described in the context of the following exemplary system and/or method.

FIG. 1 illustrates an exemplary system for behavioral assessment of a user, according to some embodiments of the present disclosure.

The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106.

The plurality of modules 106 include programs or coded instructions that supplement applications or functions performed by the system 100 for executing different steps involved in the process of behavioral assessment, being performed by the system 100. The plurality of modules 106, amongst other things, can include routines, programs, objects, components, and data structures, which performs particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for the behavioral assessment.

The data repository (or repository) 110 may include a plurality of abstracted piece of code for refinement and data that is processed, received, or generated as a result of the execution of the plurality of modules in the module(s) 106.

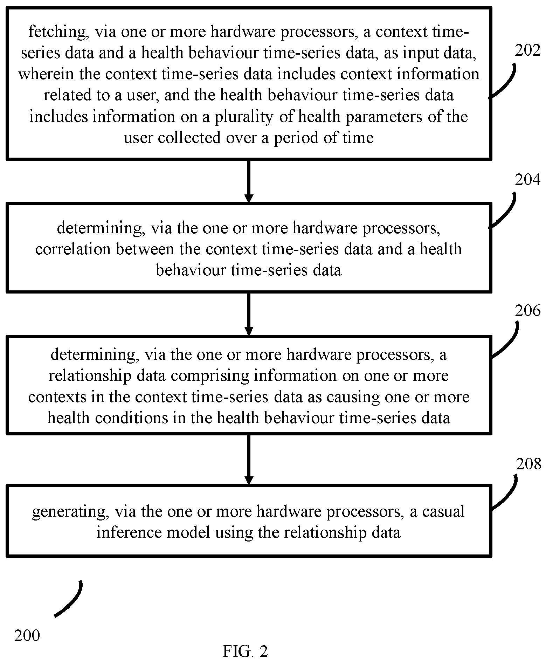

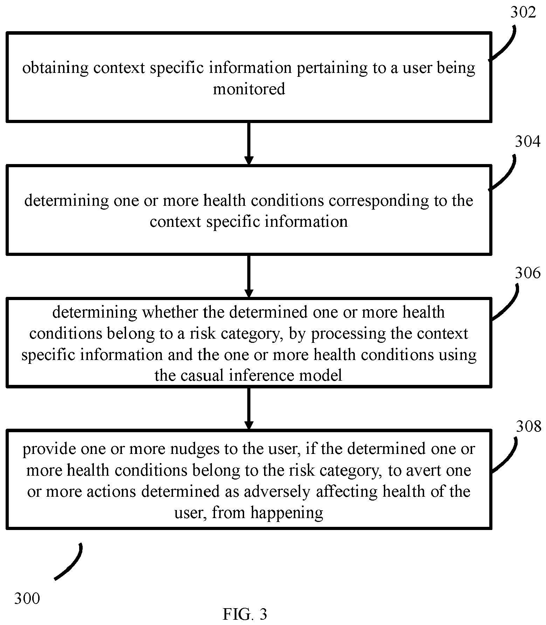

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to the steps in flow diagrams in FIG. 2 and FIG. 3.

FIG. 2 is a flow diagram depicting steps involved in the process of generating a causal inference model for behavioral assessment of diagram according to some embodiments of the present disclosure.

In an embodiment, the system 100 comprises one or more data storage devices or the memory 104 operatively coupled to the processor(s) 102 and is configured to store instructions for execution of steps of the method 200 by the processor(s) or one or more hardware processors 102. The steps of the method 200 of the present disclosure will now be explained with reference to the components or blocks of the system 100 as depicted in FIG. 1, the steps of flow diagram as depicted in FIG. 2, and the example architecture in FIG. 3. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of the method 200, the system 100 fetches, via the one or more hardware processors 102, a context time-series data and a health behaviour time-series data, as input data, wherein the context time-series data includes context information related to a user, and the health behaviour time-series data includes information on a plurality of health parameters of the user collected over a period of time. Further, the context information may include data such as but not limited to a) location in which the user is present, b) one or more other users in vicinity of the user, c) one or more activities being performed by the user, and d) one or more situational information pertaining to the user. Similarly, the health behaviour time-series data may include data that represents health condition of the user, in terms of various health parameters such as but not limited to blood pressure, heart rate, and body temperature.

Further, at step 204 of the method 200, the system 100 determines, via the one or more hardware processors, correlation between the context time-series data and a health behaviour time-series data. In an embodiment, the system 100 determines the correlation in terms of time, i.e. the system 100 correlates the health behaviour time-series data containing information on one or more health parameters being monitored, at an instance, with the context (for example, a location, an activity, and so on) that is determined at the same instance.

Further, at step 206 of the method 200, the system 100 determines, via the one or more hardware processors 102, a relationship data comprising information on one or more contexts in the context time-series data as causing one or more health conditions in the health behaviour time-series data. For example, consider that the location of the user at an instance of monitoring indicates that the user is at a gym. As the user maybe working out, there maybe a spike in heart rate of the user. The system 100 relates the spike in heart rate, to the presence of the user at the gym and one or more activities (which in this case maybe exercise) being performed by the user. The relationship data thus generated, indicates to the system 100 that the workouts at the gym causes the rise in heart rate of the user. In similar manner, other contexts are related to the corresponding health parameters, and the relationship data is determined.

Further, at step 208 of the method 200, the system 100 generates, via the one or more hardware processors 102, a casual inference model using the relationship data. The system 100 may use any suitable machine learning approach to train and generate the casual inference model, by using the relationship data as training data. The casual inference model is a reverse causality model facilitating mapping of context with one or more behaviour of the user.

The casual inference model maybe further used for processing real-time data to perform the behavioral analysis. In an embodiment, in the context of the embodiments disclosed herein, the term `behavioral analysis' refers to a process of determining, based on an identified context of a user, whether the user tends to perform any action that may adversely affect health of the user, and then executing an action to prevent the action from being performed/executed. Steps involved in this process are depicted in method 300 in FIG. 3, and are explained hereafter in detail.

At step 302 of the method 300, the system 100 obtains context specific information pertaining to a user being monitored. The context specific information may indicate a current location of the user, and/or an activity being performed by the user. The system 100 may obtain the context specific information using appropriate means. For example, the location of the user may be identified from tracked location data of mobile phone of the user. Upon receiving the location data, the system 100 is configured to further interpret the obtained location data to determine where exactly the user is present (for example, a gym, a hospital, a smoking zone of office, and so on). Further, at step 304 of the method 300, the system 100 determining one or more health conditions corresponding to the context specific information, from one or more health monitoring sensors. For example, the user maybe wearing a wearable device (such as a smart watch), which has sensors to monitor and collect details on heart rate, blood pressure and so on. The system 100 may be configured to be in communication with the wearable device to collect information on various health parameters, which may together indicate/represent a health condition of the user. For example, a high value of heart rate and blood pressure may be indicative of high chances of heart related diseases, stroke, and so on. In another embodiment, the term `health condition', in the context of the embodiments disclosed herein, may also refer to a probability of the user being involved in an activity that is potentially dangerous to health of the user. For example, consider that the identified context indicates that the user is in vicinity of a smoking zone in a building (for example, office), and that the user is in company of a second user who has been tagged as a smoker, then the system 100 determines that there is a high probability of the user being involved in an active/passive smoking session, and in this case, determines and flags the health condition of the user as belonging to a risk category at step 306 of the method 300. In the aforementioned example, if the user had been in company of a non-smoker instead of the user tagged as a smoker, then the casual inference model may have marked the health condition of the user as not belonging to the risk category, as being in proximity of the smoking zone alone may not have prompted the user to smoke. Similarly, depending on various combinations of the contexts and health condition, the system 100 accordingly categorizes and determines further steps. Conditions/criteria for such interpretation and categorization are learned by the casual inference model and in turn the system 100, from method 200, by using data pertaining to different users and different health and context specific data as the training data.

If at step 306 of the method 300 the system 100 categorizes the user as belonging to the risk category, then in order to avert the determined one or more contexts adversely affecting the health condition of the user from happening, the system 100 provides one or more nudges to the user at step 308 of the method 300. The nudge maybe in a suitable form, for example, as vibration of a mobile phone or of a device which is an implementation of the system 100, or any other device that is in communication with the system 100. In an embodiment, the system 100 is configured to provide a pre-defined number of the nudges, at pre-defined intervals. In another embodiment, after providing a first nudge the system 100 continues to monitor context and health specific data of the user, and determines if the user has positively responded to the nudge. In the context of the embodiments disclosed herein, positively responding may refer to any action that averts the determined one or more contexts adversely affecting the health condition of the user. In the context of the aforementioned example, positively responding to the nudge may be by moving away from the smoking zone or the second user who has been tagged as a smoker.

In an embodiment, for this example scenario to be practical, the system 100 requires a) location of the second user, and b) information that the second user is a smoker. For this purpose, it may be required that the second user also is a user of the system 100, and has registered a user profile containing certain personal information, with the system. The personal information in this example implementation may contain data on habits of the user (such as smoking habits, drinking habits, exercise habits and so on), along with other details such as but not limited to name, age, sex, and so on. This mode of implementation may build a user community, from whom the system 100 can collect context specific and health specific data, for the behavioral analysis.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problem of behavioral analysis. The embodiment, thus provides a mechanism for generating a casual inference model, which is a reverse causality model facilitating mapping of context with one or more behaviour of the user. Moreover, the embodiments herein further provide a mechanism to collect and process real-time data using the casual inference model, to perform behavioral analysis of the user.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200), comprising:
fetching (202), via one or more hardware processors, a context time-series data and a health behaviour time-series data, as input data, wherein the context time-series data includes context information related to a user, and the health behaviour time-series data includes information on a plurality of health parameters of the user collected over a period of time;
determining (204), via the one or more hardware processors, correlation between the context time-series data and a health behaviour time-series data;
determining (206), via the one or more hardware processors, a relationship data comprising information on one or more contexts in the context time-series data as causing one or more health conditions in the health behaviour time-series data; and
generating (208), via the one or more hardware processors, a casual inference model using the relationship data.

2. The method as claimed in claim 1, wherein the context information is determined in terms of at least one of a) location in which the user is present, b) one or more other users in vicinity of the user, c) one or more activities being performed by the user, and d) one or more situational information pertaining to the user.

3. The method as claimed in claim 1, comprising:
obtaining (302) context specific information pertaining to a user being monitored;
determining (304) one or more health conditions corresponding to the context specific information;
determining (306) whether the determined one or more health conditions belong to a risk category; and
providing (308) one or more nudges to the user, if the determined one or more health conditions belong to the risk category, to avert one or more actions determined as adversely affecting health of the user, from happening.

4. The method as claimed in claim 1, wherein the casual inference model is a reverse causality model facilitating mapping of context with one or more behaviour of the user.

5. A system (100), comprising:
one or more hardware processors (102);
a communication interface (112); and
a memory (104) storing a plurality of instructions, wherein the plurality of instructions when executed, cause the one or more hardware processors to:
fetch a context time-series data and a health behaviour time-series data, as input data, wherein the context time-series data includes context information related to a user, and the health behaviour time-series data includes information on a plurality of health parameters of the user collected over a period of time;
determine correlation between the context time-series data and a health behaviour time-series data;
determine a relationship data comprising information on one or more contexts in the context time-series data as causing one or more health conditions in the health behaviour time-series data; and
generate a casual inference model using the relationship data.

6. The system as claimed in claim 5, wherein the one or more hardware processors are configured to determine the context information in terms of at least one of a) location in which the user is present, b) one or more other users in vicinity of the user, c) one or more activities being performed by the user, and d) one or more situational information pertaining to the user.

7. The system as claimed in claim 5, wherein the one or more hardware processors are configured to:
obtain context specific information pertaining to a user being monitored;
determine one or more health conditions corresponding to the context specific information;
determine whether the determined one or more health conditions belong to a risk category, by processing the context specific information and the one or more health conditions using the casual inference model; and provide one or more nudges to the user, if the determined one or more health conditions belong to the risk category, to avert one or more actions determined as adversely affecting health of the user, from happening.

8. The system as claimed in claim 5, wherein the casual inference model is a reverse causality model facilitating mapping of context with one or more behaviour of the user.

9. One or more non-transitory machine-readable information storage mediums comprising one or more instructions which when executed by one or more hardware processors cause:
fetching a context time-series data and a health behaviour time-series data, as input data, wherein the context time-series data includes context information related to a user, and the health behaviour time-series data includes information on a plurality of health parameters of the user collected over a period of time;
determining correlation between the context time-series data and a health behaviour time-series data;
determining a relationship data comprising information on one or more contexts in the context time-series data as causing one or more health conditions in the health behaviour time-series data; and
generating a casual inference model using the relationship data.

10. The one or more non-transitory machine-readable information storage mediums as claimed in claim 9, wherein the context information is determined in terms of at least one of a) location in which the user is present, b) one or more other users in vicinity of the user, c) one or more activities being performed by the user, and d) one or more situational information pertaining to the user.

11. The one or more non-transitory machine-readable information storage mediums as claimed in claim 9, comprising:
obtaining context specific information pertaining to a user being monitored;
determining one or more health conditions corresponding to the context specific information;
determining whether the determined one or more health conditions belong to a risk category; and
providing one or more nudges to the user, if the determined one or more health conditions belong to the risk category, to avert one or more actions determined as adversely affecting health of the user, from happening.

12. The one or more non-transitory machine-readable information storage mediums as claimed in claim 9, wherein the casual inference model is a reverse causality model facilitating mapping of context with one or more behaviour of the user.
